# FASCICULE DE BREVET EUROPEEN

(11) **EP 0 540 413 B1**
(45) Date de publication et mention de la délivrance du brevet: **17.07.1996**
(21) Numéro de dépôt: 92402923.4
(22) Date de dépôt: 28.10.1992
(51) Int. Cl.: A61K 9/16, A23K 1/18

(54) **Système véhicule de principe actif ou de pigment permettant l'administration par voie orale chez les poissons et crustacés**
System zur Verabreichung von Wirkstoffen oder Pigmente an Fische oder Krebstiere
Vehicle system for an active or a dye allowing an orale administration to fish or to crustaceans

(30) Priorité: 30.10.1991 FR 9113419
(43) Date de publication de la demande: 05.05.1993
(73) Titulaire: Maingault, Philippe, F-49260 Saint Macaire du Bois (FR)
(72) Inventeur: Maingault, Philippe, F-49260 Saint Macaire du Bois (FR)
(74) Mandataire: Dawidowicz, Armand

(56) Documents cités:
- EP-A- 0 357 793
- EP-A- 0 389 347
- WO-A-81/00812
- WO-A-85/05015
- AU-A- 495 075
- GB-A- 2 144 331
- US-A- 4 401 456
- J. OF PHARMACEUTICAL SCIENCES vol. 78, no. 11, Novembre 1989, WASHINGTON pages 964 - 967 BODMEIER R. ET AL 'SPHERICAL AGGLOMERATES OF WATER-INSOLUBLE DRUGS'
- PHARMACEUTICAL RESEARCH vol. 8, no. 3, 1991, NEW-YORK LONDON pages 341 - 344 KWOK K.K. ET AL 'PRODUCTION OF 5-15 MICROM. DIAMETER MICROCAPSULES BY AN AIR ATOMIZATION TECHNIQUE'
- PATENT ABSTRACTS OF JAPAN vol. 100, no. 67 15 Mars 1986
- CHEMICAL ABSTRACTS, vol. 110, no. 21, 22 Mai 1989, Columbus, Ohio, US; abstract no. 190936v, PEDERSEN S.S. ET AL 'Purification, characterization, and immunological cross-reactivity of alginates produced by mucoid Pseudomonas aeruginosa from patients with cystic fibrosis"
- CHEMICAL ABSTRACTS, vol. 107, no. 6, 3 Août 1987, Columbus, Ohio, US; abstract no. 38386v, 'Growth-hormon-containing feeds for fish' page 576 ;colonne 2 ;
- DATABASE WPIL Section Ch, Derwent Publications Ltd., London, GB; Class B, AN 88354453
- CENTRAL PATENTS INDEX, BASIC ABSTRACTS JOURNAL Section Ch, Derwent Publications Ltd., London, GB; Class C, AN 8018957C

## Description

La présente invention concerne une préparation administrable par voie orale chez les poissons et crustacés du type incorporant un principe actif ou un pigment assimilable par l'organisme desdits poissons ou crustacés au niveau de l'extrémité distale du tube digestif ainsi qu'un procédé de fabrication de ladite préparation et l'utilisation d'un alginate purifié pour l'obtention de cette préparation.

Le développement de grands élevages dans le domaine de l'aquaculture oblige aujourd'hui les éleveurs à pratiquer un suivi médical très performant en raison des sommes d'argent mises en jeu. Plusieurs possibilités leur sont offertes mais le plus souvent, ils préfèrent prévenir que guérir et utilisent donc des méthodes telles que la vaccination. En effet, la prévention permet de limiter le recours à des traitements lourds qui sont notamment réalisés par dissémination d'antibiotiques dans le milieu de culture des poissons et crustacés. Or, une augmentation de l'utilisation des antibiotiques engendre inévitablement une augmentation de la résistance des bactéries à ces antibiotiques, ce qui à terme impliquera que bon nombre d'entre eux seront inefficaces. De plus, dans le cas de maladies virales, la prévention par le biais de la vaccination demeure la seule thérapie efficace.

Cependant, pour que la prévention soit pratiquée de manière systématique par les éleveurs, elle doit être simple à mettre en oeuvre, efficace, bon marché et ne pas nécessiter des manipulations importantes par l'éleveur. Or, aujourd'hui, on constate que prévention est synonyme de vaccination et que cette dernière se pratique par balnéation ou éventuellement injection (méthode plus théorique que pratique). La balnéation réalisée en bac ne s'applique qu'aux juvéniles et ne protège pas l'animal durant toute sa vie en élevage. Quant à l'injection, elle est extrêmement lourde puisqu'elle implique de piquer les poissons un à un. Dans tous les cas, ces méthodes provoquent un stress très important des animaux avec des retards de croissance, l'apparition de maladies opportunistes et éventuellement la mort. En conséquence, le mode d'administration de ces vaccins est loin d'être performant. Pourtant la vaccination présente un avantage très important. Des formes galéniques très élaborées telles que la microencapsulation permettent de cibler parfaitement l'organe que le principe actif doit atteindre. Ainsi un article du "pharmaceutical research", vol 8, N° 3, 1991, pages 341 à 344 décrit la préparation de microcapsules alginate-polylysine intégrant le bacille Calmette-Guérin en vue d'une administration de ce dernier par inhalation. La technique de vaccination décrite dans cette publication n'est en aucun cas transposable à des élevages en masse de poissons ou de crustacés notamment en raison de son mode d'administration (inhalation) et de la forme galénique qui répond parfaitement aux caractéristiques du système respiratoire et immunitaire humain qui diffère grandement du système digestif et immunitaire du poisson. Le brevet GB-A-2.144.331 décrit quant à lui une solution immunogène administrable oralement comprenant des protozoaires parasites à l'état sporulé enveloppés à l'intérieur d'une matrice se présentant sous forme de gel à partir de laquelle les protozoaires sont libérés lors de l'injection chez un hôte. Ce brevet concerne donc une composition susceptible d'incorporer un principe actif s'administrant oralement, ledit principe actif étant intégré à l'intérieur d'une matrice polysaccharidique bioérodable. Cette composition, bien que s'appliquant aux animaux ne peut en aucun cas être transposée aux poissons et crustacés d'une part en raison de la taille des particules qui sont proposées (3 à 5 mm), d'autre part en raison du fait qu'on utilise des alginates relativement grossiers non purifiés. Ainsi par exemple le manucol LB décrit dans l'exemple utilisé comme alginate de base et constituant la base du gel ne peut en aucun cas être utilisé dans la composition et le procédé objet de l'invention. En outre, les éléments incorporés devant induire une réponse immunitaire au niveau de l'organisme de l'animal sont des éléments à l'état sporulé qui présentent là encore des tailles non compatibles lorsqu'on travaille chez les poissons ou les crustacés.

Le but de la présente invention est donc de proposer une nouvelle préparation administrable par voie orale chez les poissons et crustacés qui n'est pas libérée dans l'environnement mais qui au contraire se libère de manière passive au niveau de l'extrémité distale du tube digestif du poisson ou du crustacé, son administration étant simple à mettre en oeuvre.

Un autre but de l'invention est de mettre au point un procédé de fabrication de cette préparation.

L'invention concerne à cet effet une préparation administrable par voie orale chez les poissons et crustacés du type incorporant un principe actif ou un pigment assimilable par l'organisme desdits poissons ou crustacés au niveau de l'extrémité distale du tube digestif, caractérisée en ce qu'elle est constituée de particules alimentaires spécifiques aux poissons et crustacés à l'intérieur desquelles sont incorporées des fractions d'un mélange d'un principe actif ou d'un pigment et d'une matrice polymérique bioérodable constituée par un alginate purifié se présentant sous forme de particules de taille inférieure à 500 µm, la matrice servant de véhicule au principe actif jusqu'à l'extrémité distale du tube digestif.

L'invention concerne également un procédé de fabrication d'une préparation administrable par voie orale, caractérisé en ce qu'on prépare une solution comprenant au moins de l'alginate de sodium purifié et un principe actif ou un pigment, en ce qu'on fractionne le mélange sous forme de gouttelettes, de particules ou de filaments de taille inférieure à 500 microns, de préférence comprises entre 5 et 100 microns, en ce qu'on réticule le mélange fractionné dans un bain de chlorure de calcium, en ce qu'on récupère les particules, gouttelettes, filaments d'alginate/principe actif ou pigment formés, et en ce qu'on incorpore les particules, gouttelettes, filaments à l'état sec ou hydraté à l'alimentation des poissons ou crustacés. On notera que ce procédé de fabrication est mis en oeuvre à froid dans un milieu non-solvant afin d'éviter une dégradation du principe actif ou du pigment.

L'invention concerne enfin un alginate purifié pour l'obtention de la préparation selon l'invention.

On connaît, par J. of Pharmaceutical Sciences, vol. 78, no. 11. 11/89, pages 964-967, un mélange d'un principe actif et d'une matrice polymérique biodégradable constituée par un alginate. Cependant, ce document prévoit une taille de particules de quelques centaines de µm, ce qui est incompatible avec une application aux poissons et crustacés, lesquels ne sont d'ailleurs pas prévus dans cette publication. Ce document ne prévoit pas l'utilisation d'alginate purifié.

D'autres caractéristiques et avantages de l'invention apparaîtront encore à la lecture de la description détaillée qui suit, laquelle description est donnée surtout à titre d'exemple.

Pour l'administration du principe actif, il convient au préalable d'intégrer le principe actif à une solution polymérique de manière à véhiculer le principe actif. La solution comprend au moins un alginate purifié et le principe actif. L'alginate est de préférence l'alginate de sodium et sa nature sera variable en fonction de la forme, de la porosité et de la résistance physico-chimique souhaitées. En effet, cet alginate peut être du type guluronique ou manuronique. Cependant, cet alginate devra impérativement être purifié avant son incorporation au principe actif. Par purification, on entend l'élimination quasi-totale des impuretés solides qui représentent généralement 1 à 3 % de matières insolubles de diamètre de l'ordre de 40 µm qui entraînent une fragilité de la matrice et surtout des bouchages au niveau du système de pulvérisation lors du fractionnement de la solution. En outre, l'alginate contient des endotoxines, en particulier des lipopolysaccharides susceptibles d'interférer au niveau de la réponse immunitaire. Ceux-ci doivent donc être au moins partiellement éliminés. Enfin, l'alginate a une charge microbiologique relativement importante supérieure à 3 000 germes/g que l'on doit éliminer par microfiltration de manière à éviter que des germes revivifiables interfèrent sur l'action du principe actif et en outre déstabilise la matrice. En conséquence, l'alginate utilisé ne peut pas être quelconque mais doit au contraire être un alginate particulièrement pur. Il en est de même en cas d'administration d'un pigment.

Le principe actif est quant à lui fonction du but de l'administration. Ainsi, à titre non limitatif, on peut citer comme principe actif, les vaccins, les médicaments, les hormones de croissance, etc.

Si on prend l'exemple d'un principe actif constitué par un vaccin, la composition de 100 ml de la solution pourra être la suivante:

| | % dans la solution (V/V) |
|---|---|
| Alginate de sodium à 3 % filtré et purifié | 50 % |
| Solution à base de fractions bactériennes/ml | 9 % |
| Eau purifiée à 20° | 41 % |

La concentration finale dans ce cas du polymère dans la solution est de 1%. En pratique, la concentration du polymère dans la solution qui sera fonction de la porosité désirée sera de préférence comprise entre 0,1 et 10 %. Quant à la viscosité de la solution, elle sera fonction de la forme désirée (microparticules, microsphère, filament, matrice pulvérisable, etc.) et sera de préférence inférieure à 5 000 ctp.

La concentration du principe actif sera fonction du type de principe actif utilisé. Par exemple, dans le cas d'un vaccin, la concentration vaccinante sera fonction du poisson (espèce, âge), de l'aliment (pâteux, sec, préparé extemporanément), du rythme de distribution des aliments, des conditions d'élevage et naturellement du type de vaccin. On constate pour un vaccin qu'il est préférable d'utiliser des fractions bactériennes ou virales plutôt que des bactéries ou des virus entiers. Dans le cas des bactéries, on utilisera en particulier des lipopolysaccharides. Par contre, dans le cas des virus, on utilisera des glycoprotéines membranaires. Ces éléments pouvant être utilisés seuls ou associés à d'autres éléments.

Lorsque la solution polymérique intégrant le principe actif est préparée, elle est introduite dans une seringue et est extrudée, c'est-à-dire forcée à travers une aiguille grâce à une force variable. Simultanément, le jet de solution est fractionné sous forme de gouttelettes à la sortie de l'aiguille soit par ultrason soit par flux d'air concentrique à l'aiguille. La pulvérisation "airless" donne également de bons résultats. Le liquide est projeté sous forme de fines gouttelettes dans un bain de chlorure de calcium. Les ions calcium jouent un rôle de réticulant de l'alginate de sodium par contact et forment un microgel avec ce dernier. Une fois les particules consolidées, elles sont récupérées sur un tamis ou par centrifugation et lavées à l'eau purifiée jusqu'à disparition des ions chlorures dans l'eau de lavage. L'élimination des ions diborures est particulièrement importante car leur présence est susceptible de dénaturer l'appétance des aliments véhiculés. Les particules obtenues peuvent alors être intégrées immédiatement à l'aliment sous forme hydratée ou être séchées à l'air ou lyophilisées préalablement à leur incorporation.

La méthode de fractionnement est particulièrement importante car elle doit permettre d'obtenir des particules dont la dimension est impérativement inférieure à 500 µm et de préférence comprise dans la plage 5-100 µm. Cette taille des particules est indispensable pour arriver à véhiculer le principe actif ou le pigment jusqu'à la partie distale du tube digestif. On constate en effet que, dans le cas de la vaccination, celle-ci n'est effective que si le principe actif est pinocyté ou phagocyté en partie terminale du tube digestif. Dans le cas d'une libération en amont de cette extrémité terminale du tube digestif, on constate une augmentation de la tolérance ou une destruction chimique du principe actif en raison d'un PH particulièrement acide au niveau de l'estomac ou une destruction biologique de ce principe actif notamment par des enzymes. Il est donc impératif que la taille des particules soit appropriée pour permettre une libération à un stade optimal du tube digestif.

On constate en outre que l'alginate incorporant le principe actif ou le pigment doit représenté moins de 20 % du poids total de la particule alimentaire qui présente généralement une taille de l'ordre de 3 à 6 mm. Si cette proportion n'est pas respectée, on observe une modification du transit digestif.

A partir de la solution polymérique décrite ci-dessus, on peut à titre d'exemple, pour mettre en oeuvre les étapes du procédé qui viennent d'être décrites, prendre une buse à projection circulaire (diamètre de l'orifice 1mm).

On pulvérise avec un débit de 16ml/mn sous une pression d'air de 1 bar. La solution est projetée dans un bain de chlorure de calcium (30 g/l) avec une hauteur de projection égale à 30 cm. Le temps de consolidation des particules est d'environ 15mm. Après lavage et égouttage, on obtient environ 410 g de préparation administrable par voie orale avec une teneur en eau de 87,7%.

Pour parfaire l'efficacité de la préparation, il est possible d'ajouter à la solution de départ un agent tensioactif non ionique tel qu'un polyoxyéthyléné ou une copolymère d'oxydes d'éthylène et propylène qui agit sur les surfaces des microparticules en les rendant plus ou moins hydrophiles de manière à influencer leur captation par les cellules.

De même, indépendamment de la taille, la forme des particules peut être adaptée de manière à faire varier la vitesse de transit dans l'organisme en fonction du type de principe actif et ou de pigment à augmenter diminuer le temps de contact avec la muqueuse. Cette forme des particules est notamment fonction de la technique de fractionnement utilisée au niveau du mélange alginate/principe actif ou de pigment avant son incorporation au chlorure de calcium. En effet, si on réalise un fractionnement du mélange par pulvérisation comme cela a été décrit ci-dessus, on aura tendance à obtenir des particules sensiblement sphériques. Si au contraire, on remplace l'étape de pulvérisation par une extrusion, on obtiendra des filaments. Enfin, si on émulsionne le mélange principe actif/alginate, on aura tendance à obtenir des billes ou des gouttelettes de dimension inférieure à celles obtenues dans le cas de la pulvérisation. Bien évidemment d'autres techniques de fractionnement peuvent être utilisées, l'émulsion, la pulvérisation ou l'extrusion n'étant citées ici qu'à titre d'exemples.

Dans le cas d'un fractionnement par émulsification du mélange solution d'alginate de sodium/principe actif, on peut pratiquer de la manière suivante :
On prépare une solution contenant :
Solution alginate de sodium 2 % : 50 ml
Vaccin : 9 x 10˝ bact/ml
Huile de vaseline fluide : 50 ml.
Copolymère OE OP 7 %.
On émulsifie par agitation à 20 000 tr/min par exemple avec un ULTRATURAX.
On ajoute 50 ml de CaCl₂ 10 % sous agitation.
On sépare par décantation et on lave les particules obtenues.
Ainsi, 1 g d'alginate + 5 g de CaCl₂ donnent 5,5 g de billes avec une teneur en eau de 95 %.

L'ensemble des paramètres décrits ci-dessus montrent que, pour chaque type de principe actif, la préparation doit être adaptée au niveau de sa composition et de son procédé de fabrication même si son procédé d'administration reste identique. En conséquence, il n'y a pas de composition type de la préparation ni de procédé de fabrication type, bien qu'un certain nombre de critères doivent être respectés.

## Revendications

1. Préparation administrable par voie orale chez les poissons et crustacés du type incorporant un principe actif ou un pigment assimilable par l'organisme desdits poissons ou crustacés au niveau de l'extrémité distale du tube digestif,
caractérisée en ce qu'elle est constituée de particules alimentaires spécifiques aux poissons et crustacés à l'intérieur desquelles sont incorporées des fractions d'un mélange d'un principe actif ou d'un pigment et d'une matrice polymérique bioérodable constituée par un alginate purifié se présentant sous forme de particules de taille inférieure à 500 µm, la matrice servant de véhicule au principe actif jusqu'à l'extrémité distale du tube digestif.

2. Préparation selon la revendication 1,
caractérisée en ce que la matrice incorporant le principe actif ou le pigment représente moins de 20 % en poids total de la particule alimentaire.

3. Préparation selon l'une des revendications 1 et 2,
caractérisée en ce que la matrice est obtenue par fractionnement par pulvérisation, extrusion ou émulsification d'une solution comprenant au moins de l'alginate de sodium purifié et le principe actif ou le pigment, ledit fractionnement devant permettre d'obtenir les particules de taille suffisamment faible, inférieure à 500 µm, puis réticulation de la solution fractionnée dans un bain de chlorure de calcium.

4. Préparation selon l'une des revendications 1 à 3,
caractérisée en ce que la concentration du polymère dans la solution est comprise de préférence entre 0,1 et 10 %.

5. Préparation selon l'une des revendications 1 à 4,
caractérisée en ce que la viscosité de la solution est de préférence inférieure à 5 000 ctp.

6. Préparation selon l'une des revendications 1 à 5,
caractérisée en ce que les particules constitutives de la solution sont des microsphères.

7. Préparation selon l'une des revendications 1 à 6,
caractérisée en ce que les particules dans la solution présentent une dimension comprise dans la plage 5-100 microns.

8. Préparation selon l'une des revendications 1 à 7,
caractérisée en ce que l'alginate est un alginate purifié partiellement exempt d'impuretés solides, d'endotoxines et de charges microbiologiques telles que des bactéries, des levures ou des champignons.

9. Préparation selon l'une des revendications 1 à 8,
caractérisée en ce que la matrice inclut en outre un agent tensioactif non ionique.

10. Procédé de fabrication d'une préparation selon l'une des revendications 1 à 9,
caractérisé en ce qu'on prépare une solution comprenant au moins de l'alginate de sodium et un principe actif ou un pigment, en ce qu'on fractionne le mélange, en ce qu'on réticule le mélange fractionné dans un bain de chlorure de calcium, en ce qu'on récupère les fractions alginate/principe actif ou pigment formées, et en ce qu'on incorpore les fractions à l'état sec ou hydraté à l'alimentation des poissons ou crustacés.

11. Utilisation d'un alginate purifie se présentant sous forme de particules de dimension inférieure à 500 µm en tant que matrice polymérique biodégradable incorporant un principe actif ou un pigment pour l'obtention d'une préparation destinée à une administration par voie orale à des poissons ou des crustacés selon l'une des revendications 1 à 9.

## Claims

1. Preparation which can be administrated orally to fish and crustaceans, of the type incorporating an active principle or a dye which can be assimilated by the body of the said fish or crustaceans at the distal end of the digestive tract, characterised in that it is comprised of food particles specific to fish and crustaceans, within which are incorporated fractions of a mixture of an active principle or of a dye and of a biodegradable polymeric matrix comprised of a purified alginate in the form of particles of less than 500µm in size, the matrix serving as a vehicle for the active principle to the distal end of the digestive tract.

2. Preparation according to claim 1, characterised in that the matrix incorporating the active principle or the dye constitutes less than 20% of the total weight of the food particle.

3. Preparation according to one of claims 1 and 2, characterised in that the matrix is obtained by fractionation by spraying, extrusion or emulsification of a solution comprising at least purified sodium alginate and the active principle or the dye, the said fractionation having to allow particles of a sufficiently small size, less than 500µm, to be obtained, then cross-linking of the fractionated solution in a bath of calcium chloride.

4. Preparation according to one of claims 1 to 3, characterised in that the concentration of the polymer in the solution is preferably between 0.1 and 10%.

5. Preparation according to one of claims 1 to 4, characterised in that the viscosity of the solution is preferably less than 5 000 ptc.

6. Preparation according to one of claims 1 to 5, characterised in that the constituent particles of the solution are microspheres.

7. Preparation according to one of claims 1 to 6, characterised in that the particles in the solution are of a size in the range 5-100 microns.

8. Preparation according to one of claims 1 to 7, characterised in that the alginate is a purified alginate partially freed of solid impurities, of endotoxins and of microbiological content such as bacteria, thrus fungi or fungi.

9. Preparation according to one of claims 1 to 8, characterised in that the matrix also includes a non-ionic surfactant.

10. Process for production of a preparation according to one of claims 1 to 9, characterised in that a solution is prepared comprising at least the sodium alginate and an active principle or a dye, in that the mixture is fractionated, in that the fractionated mixture is cross-linked in a bath of calcium chloride, in that the alginate/active principle or dye fractions formed are recovered and in that the fractions are incorporated in the dry or hydrated state into the food for fish or crustaceans.

11. Use of a purified alginate in the form of particles less than 500µm in size as a biodegradable polymeric matrix incorporating an active principle or a dye to obtain a preparation intended for oral administration to fish or crustaceans according to one of claims 1 to 9.

## Patentansprüche

1. Präparat zur oralen Verabreichung an Fische oder Krebstiere, das einen Wirkstoff oder ein Pigment enthält, der bzw. das durch den Organismus der besagten Fische oder Krebstiere im Bereich des distalen Endes des Verdauungskanals assimilierbar ist , **dadurch gekennzeichnet,** daß es aus spezifischen Nährstoffteilchen für Fische und Krebstiere besteht, in denen Fraktionen einer Mischung aus einem Wirkstoff oder eines Pigments und einer biologisch abbaubaren Polymergrundmasse enthalten sind, die aus einem gereinigten Alginat in Form von Teilchen mit einer Größe unter 500 µm besteht, wobei die Grundmasse als Trägersubstanz für den Wirkstoff bis zum distalen Ende des Verdauungskanals dient.

2. Präparat nach Anspruch 1 , **dadurch gekennzeichnet,** daß die Grundmasse, die den Wirkstoff oder das Pigment enthält, mindestens 20 % des Gesamtgewichts des Nährstoffteilchens ausmacht.

3. Präparat nach einem der Ansprüche 1 oder 2 , **dadurch gekennzeichnet,** daß die Grundmasse mittels Fraktionierung durch Zerstäuben, Extrudieren oder Emulgieren einer Lösung gewonnen wird, die wenigstens gereinigtes Natriumalginat und den Wirkstoff oder das Pigment enthält, wobei die besagte Fraktionierung die Gewinnung von Teilchen mit ausreichend geringer Größe, unter 500 µm, ermöglichen muß, mit anschließender Vernetzung der fraktionierten Lösung in einem Calciumchloridbad.

4. Präparat nach einem der Ansprüche 1 bis 3 , **dadurch gekennzeichnet,** daß die Konzentration des Polymers in der Lösung vorzugsweise zwischen 0,1 und 10 % liegt.

5. Präparat nach einem der Ansprüche 1 bis 4 , **dadurch gekennzeichnet,** daß die Viskosität der Lösung vorzugsweise unter 5.000 ctp liegt.

6. Präparat nach einem der Ansprüche 1 bis 5 , **dadurch gekennzeichnet,** daß es sich bei den in der Lösung enthaltenen Teilchen um Mikrosphären handelt.

7. Präparat nach einem der Ansprüche 1 bis 6 , **dadurch gekennzeichnet,** daß die in der Lösung enthaltenen Teilchen eine Größe im Bereich 5-100 Mikron aufweisen.

8. Präparat nach einem der Ansprüche 1 bis 7 , **dadurch gekennzeichnet,** daß es sich bei dem Alginat um ein gereinigtes Alginat handelt, das partiell frei von Feststoffverunreinigungen, Endotoxinen und mikrobiologischen Belastungen, wie etwa Bakterien, Hefen oder Pilzen, ist.

9. Präparat nach einem der Ansprüche 1 bis 8 , **dadurch gekennzeichnet,** daß die Grundmasse außerdem ein nichtionisches Tensid enthält.

10. Herstellungsverfahren für ein Präparat nach einem der Ansprüche 1 bis 9 , **dadurch gekennzeichnet,** daß eine Lösung zubereitet wird, die wenigstens Natriumalginat und einen Wirkstoff oder ein Pigment enthält, daß die Mischung fraktioniert wird, daß die fraktionierte Mischung in einem Calciumchloridbad vernetzt wird, daß die gebildeten Fraktionen aus Alginat/Wirkstoff oder Pigment aufgenommen werden und daß die Faktionen in trockenem oder wässerigem Zustand der Nahrung der Fische oder Krebstiere zugesetzt werden.

11. Verwendung eines gereinigten Alginats in Form von Teilchen mit einer Größe unter 500 µm als biologisch abbaubare Polymergrundmasse, die einen Wirkstoff oder ein Pigment enthält, zur Gewinnung eines Präparats für eine orale Verabreichung an Fische oder Krebstiere nach einem der Ansprüche 1 bis 9.
